# EUROPEAN PATENT APPLICATION

(11) **EP 4 836 449 A1**
(43) Date of publication of application: **04.11.2026**
(21) Application number: 26175392.5
(22) Date of filing: 29.04.2026
(51) Int. Cl.: H04R 25/00

(54) **AUDIO DEVICE AND STIMULUS ESTIMATION METHOD**

(30) Priority: 02.05.2025 EP 25173968
(71) Applicant: Interacoustics A/S, 5500 Middelfart (DK)
(72) Inventor: VAN HAUEN, Sigurd, DK-5500 Middelfart (DK); NØRGAARD, Kren Monrad, DK-5500 Middelfart (DK)
(74) Representative: Demant

(57) **Abstract**

A method (400) is disclosed, the method comprising: based on an ear-canal sound pressure (140) and an ear-simulator sound pressure (330), determining (410) whether to perform a first estimation method or a second estimation method; based on said determining (410), estimating (420, 430) a sound pressure (230) at a tympanic membrane (220) produced by at least one speaker (114) of an audio device (100) based on the first estimation method or the second estimation method.

## Description

### FIELD

The present disclosure relates to audio devices. More particularly, the disclosure relates to ear probes and a method of estimating a sound pressure at the tympanic membrane.

### BACKGROUND

Audio devices, such as ear probes used in hearing diagnostics, may be used to produce a specified sound pressure at a tympanic membrane of a patient. For example, in some hearing-diagnostic applications, the function of a patient's inner ear can be assessed by stimulating a patient's tympanic membrane by a specified sound pressure and measuring certain responses, such as evoked otoacoustic emissions (OAE), from the patient's hair cells in the cochlea. To accurately and reproducibly quantify said responses, it is necessary to accurately stimulate the tympanic membrane. Furthermore, to conform to the current OAE standard (IEC 60645-6, 2022), the stimulus levels have to be validated in an occluded-ear simulator according to IEC 60318-4 (2010) within ± 1.5 dB.

To this end, the sound pressure may depend on a voltage which is applied to a speaker of the audio device. However, the sound pressure produced at the tympanic membrane by the speaker may further depend on a size and/or geometrical conditions of the ear canal, which may differ significantly for different patients. Therefore, to stimulate the patient's tympanic membrane with a specific sound pressure, the speaker may first have to be individually calibrated to the ear of the patient. In particular, it may be necessary to determine the in-situ speaker sensitivity, i.e., the ratio of sound pressure at the tympanic membrane to voltage applied to the speaker. Assuming linearity, this may be achieved by measuring the sound pressure produced at the tympanic membrane for a single specific applied voltage.

However, direct measurements of the sound pressure at a tympanic membrane across a wide range of frequencies is inherently difficult and are not feasible with an ear probe placed in the ear canal. Thus, in many applications, the sound pressure at the tympanic membrane may have to be estimated. However, for some applications (e.g., some hearing-diagnostic applications such as evoked OAE measurements), conventional simple stimulus estimation methods (i.e., that do not require preliminary calibration of the ear probe to determine its equivalent acoustic source parameters) can typically only provide accurate stimuli in the IEC 60318-4 (2010) coupler while not accounting for variations in ear-canal geometry, thus producing significant stimulus errors in the human ear canal. Therefore, there is a need to provide an improved stimulus-estimation method that does not rely on complex ear-probe calibration methods. The present disclosure provides at least an alternative to the prior art.

### SUMMARY

To determine the sound pressure at the tympanic membrane based on a voltage applied to the speaker in situ, stimulus estimation methods based on ear-canal reflectance may be used. While these methods based on ear-canal reflectance allow for high precision in stimuli, they require a preliminary equivalent acoustic source calibration of the ear probe to determine, e.g., the ear-probe Thévenin-equivalent parameters. These methods may require more computational resources and may impose stricter requirements to acoustic and mechanical properties of the ear probe. Screening and diagnostic instruments, for which the cleanability of the ear-probe tip is of greater importance than measurement reproducibility, may render Thévenin-equivalent calibrations infeasible.

Further, a conventional method estimates the sound pressure at the tympanic membrane from the sound pressure measured by a microphone of the audio device, e.g., of an ear probe. However, at some frequencies, in particular near quarter-wave anti-resonance frequencies, the sound pressure measured by a microphone of the audio device may differ significantly from that at the tympanic membrane and result in significant stimulation errors at the tympanic membrane. This method cannot conform to IEC 60645-6 (2022) but exists in many instruments conforming to the previous OAE standard (IEC 60645-6, 2009).

According to a further conventional estimation method, the audio device (e.g., the ear probe) may first be calibrated in an occluded-ear simulator, e.g., according to IEC 60318-4 (2010), to determine a speaker sensitivity, i.e., the ratio of sound pressure at the ear-simulator reference microphone, simulating the tympanic membrane, to voltage applied to the speaker. The sound pressure at a patient's tympanic membrane may then simply be estimated based on the ear-simulator speaker sensitivity. However, while conforming to IEC 60645-4 (2022), for ear canal geometries (e.g., size and shape) that vary from the geometry of the occluded-ear simulator, this stimulus estimation method may introduce large stimulus errors.

According to a first aspect, a method is provided. The method comprises, based on a comparison of an ear-canal sound pressure and an ear-simulator sound pressure, determining whether to perform a first estimation method or a second estimation method. The method comprises, based on said determining, estimating a sound pressure at a tympanic membrane produced by at least one speaker of an audio device based on the first estimation method or the second estimation method.

According to a second aspect, an audio device is provided. The audio device is configured to perform a method according to the first aspect.

As mentioned, the method comprises estimating a sound pressure at a tympanic membrane produced by at least one speaker of an audio device. To this end, the audio device may be an ear probe. In other words, the audio device may be arranged at least partly in an ear canal of a patient. The audio device may comprise at least one microphone. The at least one speaker and/or the at least one microphone may be provided inside the audio device.

A method may be provided. The method may comprise determining an ear-simulator sound pressure of an audio device, determining an ear-canal sound pressure of the audio device, determining whether to perform a first estimation method or a second estimation method of a sound pressure at a tympanic membrane produced by at least one speaker of the audio device based on the ear-simulator sound pressure, the ear-canal sound pressure, and an ear canal measurement offset, and estimating the sound pressure at the tympanic membrane based on the first estimation method or the second estimation method.

Determining may refer to estimating and/or calculating.

Determining may refer to retrieving and/or receiving, e.g., from a memory or an external device. For example, determining an ear-simulator sound pressure of an audio device may refer to receiving said sound pressure (e.g., a pre-determined sound pressure) from a memory.

The audio device may further comprise a probe tip. The probe tip may individually couple the at least one speaker and/or the at least one microphone to the ear canal (of a user and/or a patient and/or a test subject). In other words, sound emitted by the at least one speaker may be emitted into the ear canal through the probe tip and/or a sound pressure at a surface of the probe tip may be measured by the at least one microphone.

The audio device may further comprise an ear tip. The ear tip may be provided with a soft rubber or foam material. Further, the ear tip may be mounted to the probe tip. The probe tip may be held in place inside the ear canal by the ear tip. In particular, the ear tip may acoustically and/or barometrically seal the ear canal.

The audio device may be used for medical purposes, e.g., as medical diagnostic tool. As described above, the audio device may be used to test a patient's ability to hear sounds or to test any other characteristic of the hearing system of the patient, such as OAEs.

As mentioned, the sound pressure may be produced by the at least one speaker. To this end, the at least one speaker may be configured to emit sound, in particular towards a tympanic membrane. The sound pressure may be adjusted by varying a voltage which is applied to the at least one speaker. For example, applying a higher voltage to the at least one speaker may result in a higher sound pressure. The at least one speaker may be a miniature speaker, e.g., an electrodynamic, balanced-armature, or piezoelectric speaker.

As described above, the method estimates the sound pressure at the tympanic membrane produced by the at least one speaker of the audio device based on the first estimation method or the second estimation method. In other words, according to the method one of two estimation methods may be selected to estimate a sound pressure at the tympanic membrane produced by the at least one speaker. Thereby, the first estimation method and/or the second estimation method may for example be based on or correspond to one of the estimation methods described above.

Further, according to the first aspect, the method includes determining whether to perform the first estimation method or the second estimation method based on an ear-canal sound pressure and an ear-simulator sound pressure. In other words, based on a comparison of an ear-canal sound pressure and an ear-simulator sound pressure, it is determined whether the first estimation method or the second estimation method is performed to estimate the sound pressure at the tympanic membrane. For example, if the ear-canal sound pressure is determined to be higher than an ear-simulator sound pressure, then the first estimation method may be used and if the ear-canal sound pressure is determined to be lower than an ear-simulator sound pressure, then the second estimation method may be used, or vice versa. If, for example, the ear-canal sound pressure is equal to the ear-simulator sound pressure, it may be possible to perform either one of the first estimation method and the second estimation method.

It was found that by estimating the sound pressure at the tympanic membrane based on the first estimation method or the second estimation method, an estimation of the sound pressure at the tympanic membrane is less likely to be affected by errors. In particular, it was found that a maximum absolute magnitude error between a true sound pressure at the tympanic membrane and said estimation for a multitude of simulated ear canal geometries is lower than, in particular on average lower than, a maximum absolute magnitude error between a true sound pressure at the tympanic membrane and an estimation obtained by a single estimation method. Thus, in other words, a maximum deviation of an estimation obtained according to the first aspect from a true sound pressure at the tympanic membrane may be less than a maximum deviation of an estimation obtained by a single estimation method from a true sound pressure at the tympanic membrane.

The method may further comprise, based on said estimating of the sound pressure at the tympanic membrane, adjusting a voltage applied to the at least one speaker to achieve a desired sound pressure at the tympanic membrane produced by the at least one speaker. In other words, the method may further comprise applying an adjusted voltage to the at least one speaker to obtain a desired sound pressure, the adjusted voltage being determined based on said estimating of the sound pressure at the tympanic membrane. Thus, after estimating the sound pressure at the tympanic membrane, the at least one speaker may be calibrated and a desired sound pressure may be produced at the tympanic membrane by the at least one speaker.

The ear-canal sound pressure may be measured by the at least one microphone of the audio device in an ear canal at a measurement frequency. In particular, the ear-canal sound pressure may be measured by the at least one microphone of the audio device at the probe tip of the audio device at a measurement frequency. The measurement frequency may be a frequency of sound, in particular of a sound emitted by the at least one speaker. Thus, the ear-canal sound pressure may be produced by the at least one speaker, in particular while the audio device is arranged at least in part in the ear canal. If the measurement frequency is low enough, in particular such that a wavelength of a corresponding sound wave is much larger than a size of the ear canal, the sound pressure may be estimated to be equal throughout the ear canal. Therefore, a measurement of the ear-canal sound pressure, e.g. at the probe tip of the audio device, may provide a good estimation of the sound pressure at the tympanic membrane for said low measurement frequencies.

In a very general manner, whenever the present application describes a sound pressure to be measured at a specific frequency, the at least one speaker may correspondingly simultaneously emit sound and/or produce a sound pressure at the specific frequency. Accordingly, whenever the present application describes a sound pressure to be measured, a voltage applied to the at least one speaker during said measurement may be measured accordingly. Thus, the voltage applied to the at least one speaker during said measurement may be connected to said measurement.

The ear-simulator sound pressure may be measured by at least one tympanic membrane reference microphone in a simulator at the measurement frequency. The simulator may be an occluded ear simulator. Thus, the simulator may be a device that may comprise similar geometrical dimensions and a similar shape as a typical human ear canal, in particular as a typical ear canal of an adult human. In particular, the simulator may be provided with at least one tympanic membrane reference microphone at a location of the simulator where a tympanic membrane would be in a typical human ear canal.

Thus, a characteristic of an audio device may be tested in said simulator by arranging the audio device in the simulator according to how the audio device would be arranged in an ear canal and by performing sound pressure measurements. Thereby, the at least one speaker of the audio device may produce a sound pressure in the simulator, and the at least one microphone and/or the at least one tympanic membrane reference microphone may perform sound pressure measurements. The ear-simulator sound pressure may therefore provide an estimation of a sound pressure at a tympanic membrane in a human's ear for a specific voltage applied to the at least one speaker wherein the at least one speaker emits sound at the measurement frequency.

The ear-simulator sound pressure may be measured at any point in time before measuring the ear-canal sound pressure. For example, the ear-simulator sound pressure may for example be measured once a year, once before a first use of the audio device in a human ear canal or immediately before each use of the audio device in a human ear canal. Data of the ear-simulator sound pressure may be stored in the audio device or in a central data storage. The data of the ear-simulator sound pressure may be accessed by the audio device whenever the method according to the first aspect may be performed. In particular, data of the ear-canal sound pressure may be compared to data of the ear-simulator sound pressure.

Thus, determining whether to perform a first estimation method or a second estimation method based on the ear-canal sound pressure and the ear-simulator sound pressure may comprise measuring the ear-canal sound pressure and comparing data of the ear-canal sound pressure with stored data of the ear-simulator sound pressure.

Said determining may be further based on an ear canal measurement offset. It was found that an offset may be present between a measurement in the simulator and a measurement in an ear canal. This offset may originate in a change of electrical characteristics of the at least one speaker and/or the at least one microphone over time, and/or in a difference in ear canal geometry and/or size from the real ear canal to the simulator.

By determining whether to perform the first estimation method or the second estimation method further based on the ear canal measurement offset, said offset may be accounted for. For example, the ear canal measurement offset may be multiplied to the ear-canal sound pressure or to the ear-simulator sound pressure. Alternatively, the ear canal measurement offset may be added to or subtracted from the ear-canal sound pressure or the ear-simulator sound pressure. Thus, with the ear canal measurement offset being accounted for, the ear-canal sound pressure may be compared to the ear-simulator sound pressure even more precisely.

The ear canal measurement offset may be based on a first offset sound pressure and a second offset sound pressure. The first offset sound pressure may be measured by the at least one microphone of the audio device in the ear canal at an offset frequency. The offset frequency is a frequency low enough for the wavelength to be significantly larger than the size of the ear canal. For example, the offset frequency may be a frequency of 226 Hz, which is a frequency conventionally used in tympanometry. However, other frequencies are possible. In particular, the offset frequency may be below 2000 Hz, in particular below 1000 Hz, in particular below 500 Hz, in particular below 300 Hz.

The second offset sound pressure may be measured by the at least one microphone of the audio device in the simulator at the offset frequency. As described above for the ear-simulator sound pressure, the second offset sound pressure may be measured once a year, once before a first use of the audio device in a human ear canal or immediately before each use of the audio device in a human ear canal. Data of the second offset sound pressure may be stored in the audio device or in a central data storage. The data of the second offset sound pressure may be accessed by the audio device whenever the method according to the first aspect may be performed. In particular, data of the first offset sound pressure may be compared to data of the second offset sound pressure.

As opposed to the ear-simulator sound pressure, the second offset sound pressure is measured by the at least one microphone. Thus, the first offset sound pressure and the second offset sound pressure are both measured by the at least one microphone, in particular while applying a same voltage to the at least one speaker, thus allowing for a comparison of the change in sound pressure from the simulator to the ear canal. Considering that, as described above, the sound pressure can be estimated to be essentially constant throughout the ear canal if the wavelength is significantly larger than the size of the ear canal, a comparison between the first offset sound pressure and the second offset sound pressure at the offset frequency allows to determine the ear canal measurement offset.

According to the first aspect, based on said determining, a sound pressure at a tympanic membrane produced by at least one speaker of an audio device is estimated based on the first estimation method or the second estimation method. The first estimation method may be based on the ear-canal sound pressure. As described above, for low frequencies with wavelengths much larger than the size of the ear canal, the sound pressure can be assumed to be constant throughout the ear canal.

The first estimation method may therefore comprise estimating the sound pressure at the tympanic membrane to correspond to the ear-canal sound pressure. In other words, the first estimation method may comprise estimating the sound pressure at the tympanic membrane to be equal to the ear-canal sound pressure. The first estimation method may provide a good estimation of the sound pressure at the tympanic membrane without requiring a Thévenin-equivalent calibration. However, it has been found that for some frequencies, in particular around a quarter-wave anti-resonance frequency, the first estimation method may produce faulty estimations.

The second estimation method may be based on an ear canal voltage applied to the at least one speaker. In other words, the second estimation method may estimate a sound pressure at the tympanic membrane produced by the at least one speaker based on the ear canal voltage applied to the at least one speaker. Thus, the at least one speaker may produce a sound pressure in the ear canal at the measurement frequency due to the ear canal voltage being applied to the at least one speaker. Thus, the second estimation method may be independent of the at least one microphone.

The second estimation method may be further based on a speaker sensitivity function. The speaker sensitivity function may describe a relation between a voltage applied to the speaker and a corresponding sound pressure produced at the tympanic membrane produced by the at least one speaker due to the voltage. The speaker sensitivity function may be based on a simulator voltage applied to the at least one speaker and on the ear-simulator sound pressure, wherein the simulator voltage is applied to the at least one speaker while the ear-simulator sound pressure is measured. For example, the second estimation method may estimate the sound pressure at the tympanic membrane to correspond to the measurement voltage multiplied by the speaker sensitivity function divided by the simulator voltage.

The second estimation method may be further based on the ear canal measurement offset. As described above, the ear canal measurement offset estimates a geometrical and/or size difference between the simulator and the ear canal. Thus, the second estimation method being further based on the ear canal measurement offset, said difference may be compensated and a more accurate estimation of the sound pressure at the tympanic membrane may be achieved by the second estimation method.

The second estimation method may provide a good estimation of the sound pressure at the tympanic membrane without requiring a Thévenin-equivalent calibration. However, it has been found that for some frequencies the second estimation method may produce faulty estimations. In particular, when acoustic properties such as input- and transfer impedance are not equal for the simulator and the ear canal, the second estimation method may produce faulty estimations.

As described above, a method comprising estimating a sound pressure at a tympanic membrane produced by at least one speaker of an audio device based entirely on one estimation method, e.g. the first estimation method or the second estimation method, may perform poorly for some circumstances, such as deviating ear-canal geometry and/or resonance frequencies. However, the method according to the first aspect may advantageously enable selecting one of two estimation methods, e.g., of the first estimation method and the second estimation method, depending on a condition, e.g., said determining, and may thus enable selecting an estimation method that performs better for said circumstances. For example, when the first estimation method performs poorly at an exemplary measurement frequency and the second estimation method performs better than the first estimation method at the exemplary measurement frequency, the method may determine to select the second estimation method and thus realize a better performance.

In some embodiments, it may be desirable to provide said estimation for multiple frequencies. Thus, the method may further comprise performing the steps of determining and estimating for multiple measurement frequencies, in particular for an entire frequency spectrum of measurement frequencies. In other words, the method according to the first aspect may be performed for multiple measurement frequencies. Thereby, the method may be performed repetitively. Further, the method may determine to perform the first estimation method for a first set of measurement frequencies of the multiple measurement frequencies and may determine to perform the second estimation method for a second set of measurement frequencies of the multiple measurement frequencies. For example, if the first estimation method may perform better than the second estimation method for the first set of measurement frequencies and the second estimation method may perform better than the first estimation method for the second set of measurement frequencies, the method according to the first aspect may perform better than performing only the first estimation method or only the second estimation method.

Thus, the first set of measurement frequencies and the second set of measurement frequencies may be determined based on an ear-canal sound pressure and an ear-simulator sound pressure for each measurement frequency of a set of measurement frequencies. In other words, each measurement frequency of a set of measurement frequencies may be determined to be either in the first set of measurement frequencies or in the second set of measurement frequencies based on an ear-canal sound pressure and an ear-simulator sound pressure for a respective measurement frequency. The sound pressure at the tympanic membrane may be estimated based on the first estimation method for the first set of sound frequencies and based on the second estimation method for the second set of sound frequencies.

A measurement frequency may be determined to be in the first set of measurement frequencies if the ear-canal sound pressure is larger than the ear-simulator sound pressure for said measurement frequency, in particular larger than the ear-simulator sound pressure multiplied by the ear canal measurement offset. Further, a measurement frequency may be determined to be in the second set of measurement frequencies if the ear-canal sound pressure is smaller than the ear-simulator sound pressure for said measurement frequency, in particular smaller than the ear-simulator sound pressure multiplied by the ear-canal measurement offset. Furthermore, a measurement frequency may be determined to be in one of the first set of measurement frequencies and the second set of measurement frequencies if the ear-canal sound pressure is essentially equal to the ear-simulator sound pressure for said measurement frequency, in particular essentially equal to the ear-simulator sound pressure multiplied by the ear canal measurement offset.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
- FIG. 1: illustrates an example of an audio device arranged in an ear canal;
- FIG. 2: illustrates an example of an audio device arranged in a simulator;
- FIG. 3: schematically shows an example of a method;
- FIG. 4: schematically shows another example of a method;
- FIG. 5: illustrates an example of ear-canal sound pressures and an example of ear-simulator sound pressures for different measurement frequencies; and
- FIG. 6: illustrates an example of stimulus errors for example methods.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer programs, or any combination thereof.

While, in the following, OAE measurements are used as a clear example and use case of the proposed method, the disclosure is not limited to specific diagnostic methods. The proposed method may equally be used for hearing aids or other audio devices.

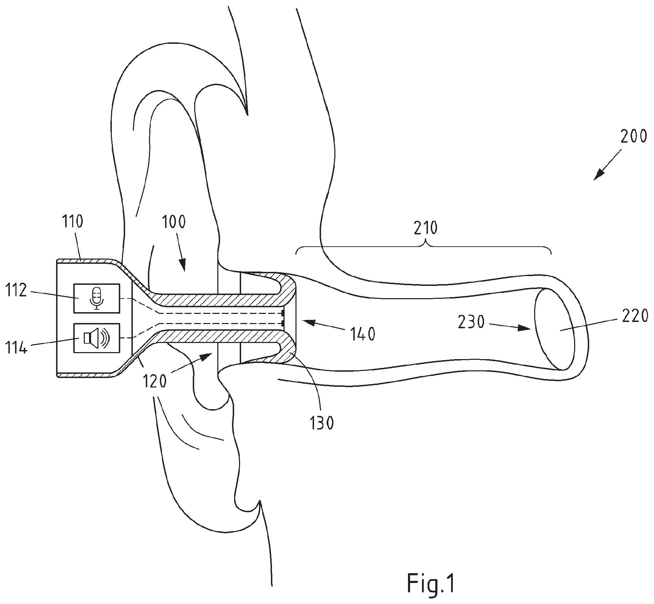

Now referring to FIG. 1, which illustrates an example of an audio device 100 arranged in part in an exemplary ear canal 200, e.g. of a user or a patient. The audio device 100 is an ear probe comprising a probe body 110 and a probe tip 120. Within the probe body 110, the ear probe comprises a microphone 112 and a speaker 114. The microphone 112 and the speaker 114 are individually connected to the probe tip 120. The probe tip 120 is connected to the ear canal 200 through the ear tip 130. The ear tip 130 is in physical contact with side walls of the ear canal. The ear tip 130 seals the ear canal from the surroundings. Sound emitted by the speaker 114 is thus emitted into the ear canal 200 via the probe tip 120.

The ear canal comprises a tympanic membrane 220 arranged at a distance from the probe tip 120. The distance corresponds to a length 210 of the ear canal 200. Sound emitted by the speaker 114 produces a sound pressure inside the ear canal 200. The speaker 114 can produce a sound pressure 230 at the tympanic membrane 220 by emitting sound into the ear canal 200. Due to reflections from the tympanic membrane 220 and the walls, the speaker also produces an ear-canal sound pressure 140 at the probe tip. The microphone 112 can measure the ear-canal sound pressure 140. The audio device 100 is arranged at least in part in the ear canal 200 such that the at least one speaker 114 and the at least one microphone 112 of the audio device 100 are arranged at least in part in the ear canal 200 and at least in part facing the tympanic membrane 220.

The microphone can also measure other sounds, e.g., otoacoustic emissions (OAE) generated by the inner ear in response to external stimuli. When sound enters the ear, it causes vibrations in the cochlea, a spiral-shaped structure in the inner ear responsible for sensing sound. These vibrations stimulate hair cells on a basilar membrane, which convert mechanical energy of the sound into electrical signals that can be processed by the brain. As a byproduct of this active process, the hair cells also emit sounds, known as otoacoustic emissions. These emissions can be measured using a sensitive microphone (e.g., the microphone 112) placed in the ear canal 200. OAEs provide valuable diagnostic information about the integrity and function of the cochlea and the outer hair cells.

OAE measurements involve measuring the response to an acoustic stimulus, conventionally using an ear probe (e.g. the audio device 100) inserted in the ear canal 200. The ear probe consists of one or more miniature speakers (e.g., speaker 114) and a microphone 112, housed in the ear-probe body 110, and individually coupled to the ear canal 200 using a probe tip 120. The ear probe is held in place by and acoustically sealed to the ear-canal walls using an ear tip 130. The ear tip 130 is usually made of a soft rubber or foam, and mounted onto the probe tip 120. The ear tip 130 also provides a barrier between the ear canal 200 and the outside world and reduces the influence of environmental noise during measurements. When using rubber ear tips 130, a barometric seal can be achieved which allows pressurizing the ear canal 200. This is needed for, e.g., tympanometry and pressurized OAE measurements.

The OAE depends on the level of the stimuli delivered by the ear probe. The level of stimuli can be referenced against or correspond to the sound pressure 230 at the tympanic membrane 220. The stimulus levels for distortion-product otoacoustic emissions (DPOAE) are defined in the OAE standard (IEC 60645-6 2022). For the stimulus levels to conform to this standard, the stimuli should be validated in an occluded-ear simulator 300 within ± 1.5 dB (IEC 60318-4, 2010).

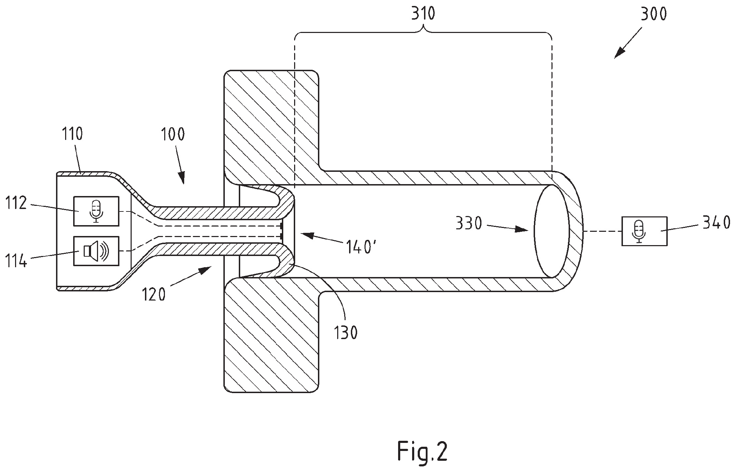

FIG. 2 illustrates an example of an audio device 100 arranged in a simulator 300, e.g., an occluded-ear simulator 300. The occluded-ear simulator 300 is designed with the acoustic transfer impedance imitating that of a typical adult ear canal 200 with a half-wavelength resonance at 13.5 kHz (± 1.5 kHz). For stimuli where the wavelength is much larger than the dimensions (e.g., the length 210) of the ear canal 200, the sound pressure is essentially uniform throughout the ear canal 200. At higher frequencies, near the quarter-wavelength anti-resonance frequency of the ear canal 200, the sound pressure can vary significantly due to standing waves between the probe tip 120 and the tympanic membrane 220. During measurements in real ears, the sound pressure 140 is recorded at the probe tip 120 and estimating the sound pressure 230 at the tympanic membrane 220 is difficult due to these standing waves.

As a conventional method for delivering stimuli to the ear, the sensitivity of the speaker 114 can be measured while the ear probe is inserted in an occluded ear simulator 300 such that the transfer impedance from ear-probe tip 120 to the tympanic membrane reference microphone 340 is embedded. This ensures that a desired sound pressure 230 at the tympanic membrane 220 can be reproduced in an occluded ear simulator 300. However, stimulus-level errors occur when the acoustic characteristics of the ear canal 200 and middle ear under test deviate from those of the occluded ear simulator 300. This method is particularly sensitive to changes around half-wave resonance frequencies and to transfer and termination impedance.

Alternatively, stimuli can be adjusted directly based on the ear-canal sound pressure 140 measured by the microphone 114. However, this can result in large errors near quarter-wave resonance frequencies. In typical ears, the stimulus-level error can be up to ± 20 dB [1]. Additionally, this approach cannot conform to the IEC 60645-6 (2022) standard.

Further alternatively, reflectance-based in-situ calibration methods, such as complex integrated pressure and propagated pressure, may enable a far more precise adjustment of the sound pressure 230 at the tympanic membrane 220 with stimulus level errors less than 1.5 dB for varying ear canal lengths 210, and thus, changing resonance quarter-wave and half-wave resonance frequencies [2]. To derive the reflectance-based quantities in situ, a prior Thévenin-equivalent calibration of the ear probe 100 is required. This requires more computational resources and imposes stricter requirements into the acoustic properties of the ear probe 100. Screening and diagnostic instruments, where the cleanability of the probe tip 120 is of greater importance, can render these Thévenin-equivalent calibration, and hereby reflectance-based calibration methods, infeasible.

Thus, an improved method is needed for more accurately delivering stimuli to the tympanic membrane 220 without requiring a Thévenin-equivalent calibration of the ear probe 100, while conforming to the OAE standard (IEC 60645-6 2022).

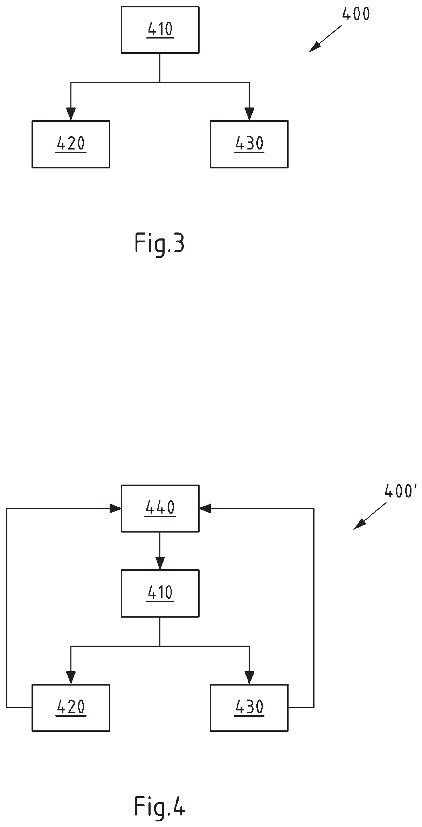

FIG. 3 schematically shows an example of a method 400. The method 400 comprises a step of determining 410 whether to perform a first estimation method or a second estimation method based on the ear-canal sound pressure 140 and the ear-simulator sound pressure 330. The method 400 further comprises a step of estimating 420, 430 the sound pressure 230 at the tympanic membrane 220 produced by the at least one speaker 114 of the audio device 100 based on the first estimation method or the second estimation method based on said determining 410. Thereby, step 420 corresponds to estimating the sound pressure 230 at the tympanic membrane 220 produced by the at least one speaker 114 of the audio device 100 based on the first estimation method and step 430 corresponds to estimating the sound pressure 230 at the tympanic membrane 220 produced by the at least one speaker 114 of the audio device 100 based on the second estimation method.

The method 400 is a hybrid in-situ calibration method that reduces the stimulus-level error at the tympanic membrane 220 of an ear under test, without being dependent on a prior Thévenin-equivalent calibration of the ear probe 100 while still conforming to the OAE standard (IEC 60645-6, 2022).

During the in-situ stimulus adjustment, only the ear-canal sound pressure 140 at the probe tip 120 can be measured. The ear-canal measurement offset represents the change in acoustic volume, and hereby the sound pressure, between the occluded ear simulator 300 and the ear canal 200 under test. The ear canal measurement offset is based on a first offset sound pressure 140 and a second offset sound pressure 140'.

The first offset sound pressure 140 is measured by the at least one microphone 112 of the audio device 100 in the ear canal 200 at an offset frequency. The second offset sound pressure 140' is measured by the at least one microphone 112 of the audio device 100 or the microphone 340 of the ear simulator 300 in the ear simulator 300 at the offset frequency. In the present embodiment, the ear-canal measurement offset is calculated by dividing the first offset sound pressure 140 by the second offset sound pressure 140'.

The ear canal measurement offset can also be obtained by dividing a first output voltage of the at least one microphone 112 by a second output voltage of the at least one microphone 112. Thereby, the first output voltage is an output voltage of the at least one microphone 112 due to the sound pressure 140 and the second output voltage is an output voltage of the at least one microphone 112 due to the sound pressure 140.

The sound pressures 140, 140' at the probe tip 120 can be calculated by dividing an output voltage of the at least one microphone 114 by the microphone sensitivity. Errors in the microphone sensitivity can directly impact the stimulus-level error when using the ear-canal sound pressure 140 as an estimate for the sound pressure 230 at the tympanic membrane 240.

The ear-canal measurement offset is calculated at a low frequency, the offset frequency, where the wavelength is much longer than the dimensions of an ear canal, e.g., at the probetone frequency of 226 Hz conventionally used in tympanometry. However, in more general terms, the offset frequency can be chosen to be below 2000 Hz, in particular below 1000 Hz, in particular below 500 Hz, in particular below 300 Hz.

Said determining 410 is further based on the ear-canal measurement offset. In particular, if the ear-canal sound pressure 140 is larger than the ear-simulator sound pressure 330 offset by the ear canal measurement offset, the sound pressure 230 at the tympanic membrane 220 is estimated based on the first estimation method. Conversely, if the ear-canal sound pressure 140 is smaller than the ear-simulator sound pressure 330 offset by the ear-canal measurement offset, the sound pressure 230 at the tympanic membrane 220 is estimated based on the second estimation method.

The first estimation method is based on the ear-canal sound pressure 140. The first estimation method estimates a transfer function from the probe tip 120 to the tympanic membrane 220 to be unity, i.e., that the ear-canal sound pressure 140 is the same as the sound pressure 230 at the tympanic membrane 220. In other words, the first estimation method estimates the sound pressure 230 at the tympanic membrane 220 to correspond to the measured ear-canal sound pressure 114 and in particular to be equal to the measured ear-canal sound pressure 114.

The second estimation method is based on an ear canal voltage applied to the at least one speaker 114. The second estimation method is further based on a speaker sensitivity function. The speaker sensitivity function is calculated by dividing an ear-simulator sound pressure 330 by a simulator voltage applied to the at least one speaker 114 while the ear-simulator sound pressure 330 is measured. In other words, the speaker sensitivity function determines how much voltage has to be applied to the speaker 114 to obtain a respective sound pressure 330 at the tympanic membrane reference microphone 340 while the audio device 100 is arranged in the simulator 300, as shown in Fig. 2.

During measurements in an ear under test the sound pressure 230 at the tympanic membrane 220 can be estimated as a product of a voltage currently applied to the speaker 114 (the ear canal voltage) and the speaker sensitivity function, assuming that the ear under test has the same acoustic properties such as input- and transfer impedance.

However, as this is known rarely to be true, the second estimation method is further based on the ear canal measurement offset. The sound pressure 230 at the tympanic membrane 220 can be estimated as a product of a voltage currently applied to the speaker 114 (the ear canal voltage), the speaker sensitivity function and the ear canal measurement offset. Thus, deviations of the acoustic properties can be accounted for by the ear canal measurement offset.

Based on said estimating of the sound pressure 230 at the tympanic membrane 220, a voltage applied to the at least one speaker 114 can be adjusted to achieve a desired sound pressure at the tympanic membrane produced by the at least one speaker.

Fig. 4, schematically shows another example of a method 400'. The method 400' shown in Fig. 4 comprises a further step 440 of performing the steps of determining 410 and estimating 420, 430 for multiple measurement frequencies, in particular for an entire frequency spectrum of measurement frequencies. In other words, the method 400' comprises performing method 400 repetitively for varying measurement frequencies. In particular, the method 400' is performed to estimate the sound pressure 230 at the tympanic membrane 220 for a multitude to measurement frequencies. In particular, the method 400' is performed to estimate the sound pressure 230 at the tympanic membrane 220 for an entire frequency spectrum of measurement frequencies. Hereby, the entire frequency spectrum may comprise discrete measurement frequencies distributed regularly along the spectrum.

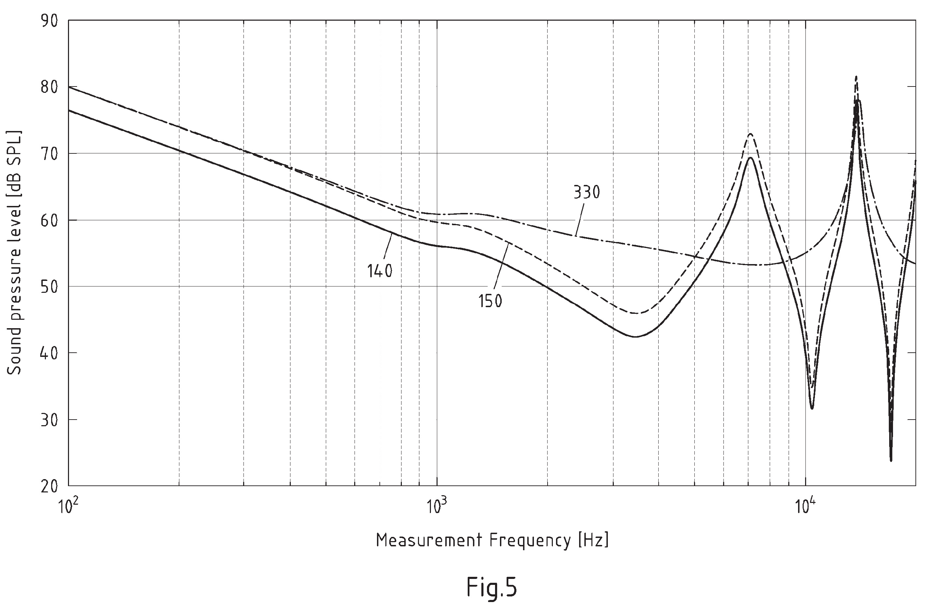

Fig. 5 illustrates an example of ear-canal sound pressures and an example of ear-simulator sound pressures for different measurement frequencies. In particular, Fig. 5 illustrates an example of ear-canal sound pressures and an example of ear-simulator sound pressures for an entire frequency spectrum of measurement frequencies. In Fig. 5, the ear-canal sound pressure 140, an offset ear-canal sound pressure 150 and the ear-simulator sound pressure 330 are shown for measurement frequencies between 100 Hz and 20.000 Hz. The offset ear-canal sound pressure 150 is based on the ear-canal sound pressure 140 and the ear canal measurement offset. In particular, the offset ear-canal sound pressure 150 is calculated by dividing the ear-canal sound pressure 140 by the ear canal measurement offset.

As can be seen in Fig. 5, for lower frequencies (e.g. below 1.000 Hz) the offset ear-canal sound pressure 150 and the ear-simulator sound pressure 330 are essentially equal. However, for frequencies above 1.000 Hz, the offset ear-canal sound pressure 150 and the ear-simulator sound pressure 330 deviate from each other up to more than 20 dB SPL.

In the example shown in Fig. 5, the sound pressure 230 at the tympanic membrane 220 is estimated based on the first estimation method, for measurement frequencies for which the offset ear-canal sound pressure 150 is higher than the ear-simulator sound pressure 330. Thus, the sound pressure 230 at the tympanic membrane 220 is estimated to be equal to the ear-canal sound pressure 140, for example for measurement frequencies between approximately 5 kHz and approximately 9 kHz.

The sound pressure 230 at the tympanic membrane 220 is estimated based on the second estimation method, for measurement frequencies for which the offset ear-canal sound pressure 150 is lower or smaller than the ear-simulator sound pressure 330. Thus, the sound pressure 230 at the tympanic membrane 220 is estimated to be equal to the product of the corresponding ear canal voltage applied to the speaker 114, the speaker sensitivity function and the ear canal measurement offset, for example for measurement frequencies below approximately 5 kHz.

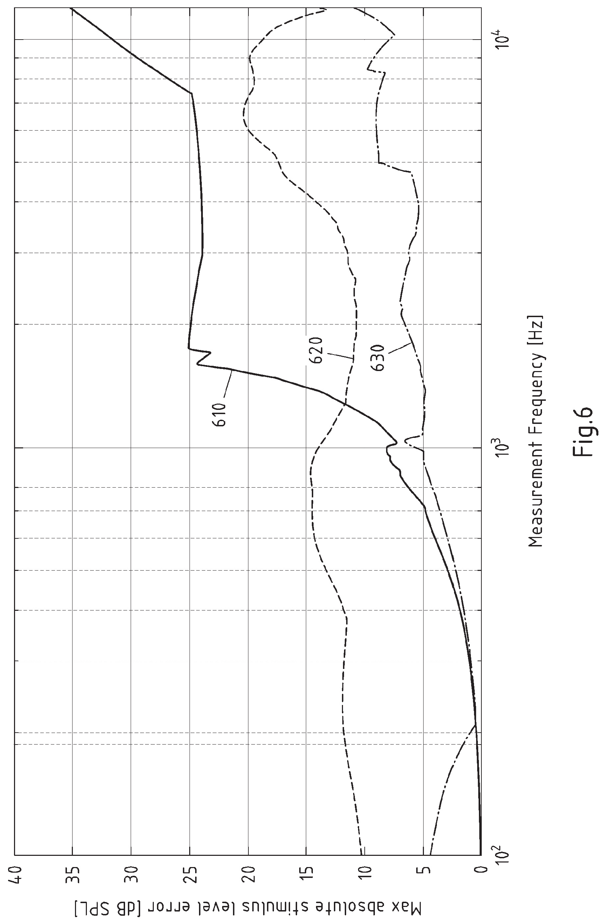

Fig. 6 illustrates an example of simulated stimulus errors for example methods. In Fig. 6, the method 400' is compared to two alternative calibration methods. The methods are compared based on their ability to estimate the sound pressure at the tympanic membrane with the stimulus error being calculated by dividing an estimated sound pressure at the tympanic membrane 220 by an actual sound pressure at the tympanic membrane 220. To quantify stimulus errors, the actual sound pressure at the tympanic membrane must be known. Measuring this quantity in human subjects is not a trivial task due to the difficulty in placing probe tube microphones close to the tympanic membrane and due to variations in the sound pressure across its surface. Instead, a variety of realistic ear canal lengths 210, ear canal cross sectional areas and tympanic membrane impedances were simulated using a transmission-line model. Figure 6 shows the maximum sound-pressure error for each of the three methods.

Shown in Fig. 6 are the maximum sound pressure error for a first test method 610, a second test method 620 and the proposed method 630. The first test method corresponds to the first estimation method as claimed herein. The second test method estimates the sound pressure 230 at the tympanic membrane 220 as a product of the ear canal voltage applied to the speaker 114 and the speaker sensitivity function.

It is evident that the stimulus-level error increases towards the quarter-wavelength anti-resonance frequency for all three methods. Further effects are also visible for all three methods when nearing the half-wavelength resonance frequency, especially for the first test method 610 where the lowest quarter-wave anti-resonance frequency simulated is 1.715 kHz. The standing-wave effects in this method make it not compliant with the OAE standard (IEC 60645-6, 2022). Furthermore, the lack of stimulus adjustment by, e.g., a low-frequency offset, is seen for the second test method 620. The proposed method 630 exhibits the overall lowest stimulus-level error among the three methods with exceptions in the range between 100 Hz and the frequency of the low-frequency offset.

According to the OAE standard (IEC 60645-6, 2022), stimulus levels are only to be validated in an occluded-ear simulator with no mention of the actual sound pressure on the tympanic membrane of the patient, nor any mention of the differences between adult and infant ear canals. This can lead to large stimulus errors due to standing waves in the ear canal, depending on the utilized stimulus-estimation method. Some devices attempt to compensate for these errors by performing various in-situ corrections. Fig. 6 shows that the known methods result in a large variation in the worst-case stimulus error across the frequency range of interest for DPOAE measurements in the simulated ears.

Although some reflectance-based calibration methods are superior to the three methods described above, e.g., complex integrated pressure and propagated pressure [2], other calibration method(s) are still relevant where device and/or ear probes do not support these reflectance-based methods. This could be due to limited computational resources or the acoustic properties of the ear probe that render these methods infeasible, e.g., in screening and diagnostic instruments where the cleanability of the probe tip is of greater importance for the clinician.

It is intended that the structural features of the devices described above, either in the detailed description and/or in the claims, may be combined with steps of the method, when appropriately substituted by a corresponding process.

As used, the singular forms "a", "an", and "the" are intended to include the plural forms as well (i.e. to have the meaning "at least one"), unless expressly stated otherwise. It will be further understood that the terms "includes", "comprises", "including", and/or "comprising", when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects. Reference to an element in the singular is not intended to mean "one and only one" unless specifically so stated, but rather "one or more". Unless specifically stated otherwise, the term "some" refers to one or more.

Accordingly, the scope should be judged in terms of the claims that follow.

### References

[1] Siegel, Jonathan H. 1994. "Ear-Canal Standing Waves and High-Frequency Sound Calibration Using Otoacoustic Emission Probes." J. Acoust. Soc. Am. 95 (5): 2589-9
[2] Norgaard, Kren Monrad, and Peter J. Bray. 2023. "Comments on Forward Pressure and Other Reflectance-Based Quantities for Delivering Stimuli to the Ear." The Journal of the Acoustical Society of America 153 (2): 909-20

## Claims

1. A method (400) comprising:
- determining an ear-simulator sound pressure (330) of an audio device,
- determining an ear-canal sound pressure (140) of the audio device,
- determining (410) whether to perform a first estimation method or a second estimation method of a sound pressure (230) at a tympanic membrane (220) produced by at least one speaker (114) of the audio device (100) based on the ear-simulator sound pressure (330), the ear-canal sound pressure (140), and an ear canal measurement offset;
- estimating (420, 430) the sound pressure (230) at the tympanic membrane (220) based on the first estimation method or the second estimation method.

2. The method (400) according to claim 1,
based on said estimating of the sound pressure (230) at the tympanic membrane (220), adjusting a voltage applied to the at least one speaker (114) to achieve a desired sound pressure (230) at the tympanic membrane (220) produced by the at least one speaker (114).

3. The method (400) according to any of claims 1 to 2,
- wherein the ear-canal sound pressure (140) is measured by the at least one microphone (112) of the audio device (100) in an ear canal at a measurement frequency, and
- wherein the ear-simulator sound pressure (330) is measured by at least one tympanic membrane reference microphone (340) in a simulator (300) at the measurement frequency.

4. The method (400) according to claim 3,
- wherein the ear canal measurement offset is based on a first offset sound pressure (140) and a second offset sound pressure (140'), and further
- wherein the first offset sound pressure (140) is measured by the at least one microphone (112) of the audio device (100) in the ear canal (200) at an offset frequency, and/or
- wherein the second offset sound pressure (140') is measured by the at least one microphone (112) of the audio device (100) in the simulator (300) at the offset frequency.

5. The method (400) according to claim 4,
wherein the offset frequency is below 2000 Hz.

6. The method (400) according to any of claims 1 to 5,
wherein the first estimation method is based on the ear-canal sound pressure (140).

7. The method (400) according to any of claims 1 to 6,
wherein the first estimation method comprises estimating the sound pressure (230) at the tympanic membrane (220) to correspond to the ear-canal sound pressure (140).

8. The method (400) according to any of claims 1 to 7,
wherein the second estimation method is based on an ear canal voltage applied to the at least one speaker (114).

9. The method (400) according to claim 8,
wherein the second estimation method is further based on a speaker sensitivity function.

10. The method (400) according to claim 9,
wherein the speaker sensitivity function is based on a simulator voltage applied to the at least one speaker (114) and on the ear-simulator sound pressure (330), wherein the simulator voltage is applied to the at least one speaker (114) while the ear-simulator sound pressure (330) is measured.

11. The method (400) according to any of claims 8 to 10,
wherein the second estimation method is further based on the ear canal measurement offset.

12. The method (400) according to any of claims 1 to 11, further comprising
- performing (440) the steps of determining (410) and estimating (420, 430) for multiple measurement frequencies.

13. An audio device (100) configured to perform a method (400) according to any of claims 1 to 12.

14. The audio device (100) according to claim 13, wherein the audio device comprises a probe tip configured to couple a speaker and a microphone of the audio device to an ear canal of a patient, wherein the speaker is configured to provide a sound pressure at a tympanic membrane of the patient in accordance with an adjustable voltage applied to the speaker.

15. The audio device (100) according to any of claims 13-14, wherein the audio device is configured to adjust the voltage applied to the speaker in response to an estimate of (420, 430) the sound pressure (230) at the tympanic membrane (220) based on a first estimation method or a second estimation method.
